# EUROPÄISCHE PATENTSCHRIFT

(11) **EP 1 171 406 B1**
(45) Veröffentlichungstag und Bekanntmachung des Hinweises auf die Patenterteilung: **21.04.2004**
(21) Anmeldenummer: 00916828.7
(22) Anmeldetag: 11.02.2000
(51) Int. Cl.: C07C 25/18, C07C 15/14

(54) **VERFAHREN ZUR HERSTELLUNG VON BIARYLEN**
METHOD OF PRODUCING BIARYLS
PROCEDE DE PREPARATION DE BI-ARYLES

(30) Priorität: 10.04.1999 DE 19916222
(43) Veröffentlichungstag der Anmeldung: 16.01.2002
(73) Patentinhaber: Degussa AG, 40474 Düsseldorf (DE)
(72) Erfinder: RIERMEIER, Thomas, D-65439 Flörsheim (DE); BELLER, Matthias, D-18119 Rostock (DE); ZAPF, Alexander, D-83024 Rosenheim (DE)
(86) Internationale Anmeldenummer: PCT/EP2000/001111
(87) Internationale Veröffentlichungsnummer: WO 2000/061531

(56) Entgegenhaltungen:
- EP-A- 0 501 268
- WO-A-93/10106
- DE-A- 4 414 499
- DE-C- 3 925 437
- US-A- 5 254 776
- CHEMICAL ABSTRACTS, Band 131, Nr. 23, 06 Dezember 1999 Columbus, Ohio, US; Zusammenfassungs-Nr. 310410b, WOLFE, J.P. et al. "A highly active catalyst for the room-temperature animation and Suzuki coupling of arylchlorides" Seite 475, Spalte 1, XP 002901169 & Angew. Chem., Int. Ed. 1999, 38(16), 2413-2416 (Eng),

## Beschreibung

Die vorliegende Erfindung betrifft ein Verfahren zur Herstellung von Biarylen mit Katalysatoren auf Basis von Palladium-Verbindungen mit Phosphitliganden

Biarylverbindungen, insbesondere Biphenytverbindungen, haben technische Bedeutung als Feinchemikalien, Zwischenprodukte für Pharmazeutika, optische Aufheller und Agrochemikalien.

Eine häufig angewandte Methode zur Synthese von Biarylen im Labormaßstab ist die Suzuki-Reaktion, bei der Iod- oder Bromaromaten und in Ausnahmefällen Chloraromaten mit Aryl-, Vinyl- oder Alkylboronsäurederivaten in Gegenwart von Palladium-Katalysatoren umgesetzt werden. Übersichtsartikel, die diese Methodik beschreiben, findet man beispielsweise in N. Miyaura, A. Suzuki, Chem. Rev. 1995, 95, 2457.
Katalysatoren, die im Rahmen der Suzuki-Reaktion verwendet werden, sind generell Palladium- und Nickelverbindungen. Trotz des ökonomischen Vorteils von Nickel-Katalysatoren (vgl. A.F. Indolese, Tetrahedron Lett. 1997, 38, 3513) werden aufgrund der geringeren Toxizität und der größeren Toleranz gegenüber funktionellen Gruppen Palladium-Katalysatoren gegenüber Nickel-Katalysatoren bevorzugt.
Im Falle des Einsatzes von Palladium-Katalysatoren werden sowohl Palladium(II)-, als auch Palladium(0)-Komplexe bei Suzuki-Reaktionen eingesetzt (vgl. M. Beller, H. Fischer, W. A. Herrmann, K. Öfele, C. Broßmer, Angew. Chem. 1995, *107*, 1992). Als katalytisch aktive Spezies formuliert man gemäß Angaben in der Literatur koordinativ ungesättigte 14- und 16-Elektronen Palladium(0)-Spezies, welche mit Donorliganden wie Phosphanen stabilisiert werden. Insbesondere beim Einsatz von kostengünstigeren Edukten wie Arylbromiden oder Arylchloriden benötigt man den Zusatz von stabilisierenden Liganden, damit eine befriedigende katalytische Aktivierung der Edukte erzielt wird.

Ein wesentlicher Nachteil der beschriebenen Suzuki-Reaktionen besteht darin, daß nur mit nicht ökonomischen Ausgangsmaterialien wie lodaromaten und aktivierten (d. h. elektronenarmen) Bromaromaten befriedigende katalytische Wechselzahlen ("tumover numbers" = TON) erzielt werden können. Ansonsten müssen bei Verwendung deaktivierter (d. h. elektronenreichen) Bromaromaten oder Chloraromaten große Mengen an Katalysator - üblicherweise 1 bis 5 mol% - zugesetzt werden, um technisch nutzbare Umsätze zu erzielen.
Aufgrund der Komplexität der Reaktionsgemische ist zudem kein einfaches Katalysatorrecycling möglich, so daß auch die Katalysatorkosten in der Regel einer technischen Realisierung entgegenstehen.
Neuere Katalysatorsysteme auf Basis wasserlöslicher Phosphane ergeben zwar für die industriell bedeutsame Umsetzung von 2-Chlorbenzonitril mit p-TolyBboronsäure befriedigende Katalysatoraktivitäten, jedoch beinhalten die Katalysatoren teure sulfonierte Phosphane. Darüber hinaus sind eine Reihe von Chloraromaten auch mit diesen Katalysatoren noch nicht technisch befriedigend zu aktivieren (vgl. S. Haber, Fine Chemical Syntheses, in B. Cornils, W. A. Herrmann, Aqueous Phase Organometallic Catalysis, Wiley-VCH: Weinheim, New York, Chichester 1998, S. 440 ff.)

Aufgabe der vorliegenden Erfindung ist die Bereitstellung eines neuen Verfahrens zur Herstellung von Biarylen, das die Nachteile der bekannten Verfahren nicht aufweist, für die großtechnische Durchführung geeignet ist und Biaryle in hoher Ausbeute, Katalysatorproduktivität und Reinheit liefern.

Diese Aufgabe wird gelöst durch ein Verfahren zur Herstellung mono-, bi- und/oder polyfunktioneller Biaryle der allgemeinen Formel (I)

Ar - Ar' (I)

in der Ar und Ar' unabhängig voneinander
- einen aromatischen Rest mit bis zu 14 Kohlenstoffatomen oder
- einen Heteroaromaten darstellen, ausgewählt aus der Gruppe der fünf-, sechsoder siebengliedrigen Ringe mit mindestens einem Stickstoff-, Sauerstoffund/oder Schwefelatom im Ring;
durch Umsetzung von Halogenaromaten der allgemeinen Formel (II)

Ar - X (II)

mit Borverbindungen der allgemeinen Formel (IIIa), (IIIb) und/oder (IIIc) 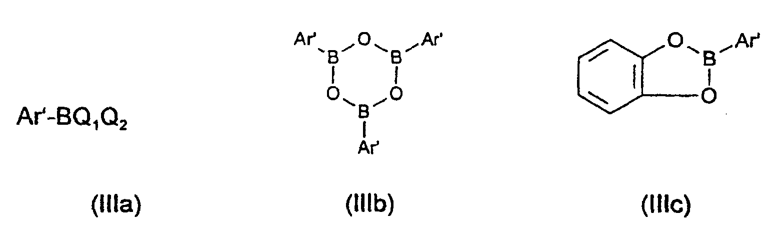 wobei in den Formeln (II), (IIIa), (IIIb) und (IIIc)
- Ar und Ar' die gleiche Bedeutung haben wie in Formel (I);
- X ausgewählt ist aus der Gruppe von Chlor, Brom, Iod, OSO₂CF₃, OSO₂Aryl-(C₆-C₁₀), OSO₂Alkyl-(C₁-C₈) und N₂⁺Y⁻, wobei Y ein Chlor-, Brom- oder lodatom oder ein Tetrafluoroborat- oder Tetraphenylboratanion darstellt;
- Q₁ und Q₂ unabhängig voneinander ausgewählt sind aus der Gruppe von OH, Fluor, Chlor, Brom, Iod, Alkyl-(C₁-C₄), Aryl-(C₆-C₁₀), Alkoxy-(C₁-C₄) und Aryloxy-(C₆-C₁₀);
in Gegenwart mindestens eines Palladiumkomplexes der allgemeinen Formel (IVa) oder (IVb), 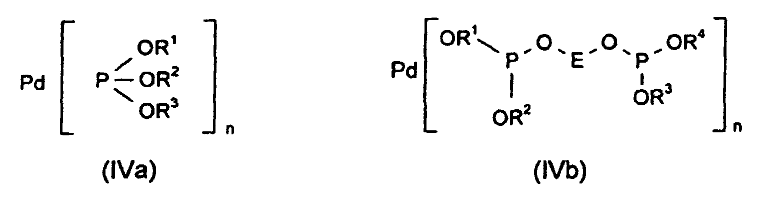 in denen
- die Reste R¹ bis R⁴ unabhängig voneinander einen (C₁-C₁₈)-Alkylrest oder einen der zuvor beschriebenen Reste Ar darstellen;
- E eine Kohlenstoffbrücke mit zwei bis sieben Kohlenstoffatomen darstellt; und
- n eine ganze Zahl von 1 bis 4 darstellt
und wobei die Palladiumkomplexe der allgemeinen Formel (IVa) oder (IVb) in situ aus mindestens einem Palladium(II)salz oder einer Palladium(0)verbindung und dem entsprechenden Phosphitliganden erhalten werden und das molare Verhältnis der eingesetzten Palladium(0)verbindung oder des Palladium (II)salzes zu eingesetztem Phosphitligand zwischen 1 : 5 und 1 : 200 liegt.

Gemäß einer weiteren Ausführungsform der Erfindung weisen
- die aromatischen Reste Ar und Ar' bis zu acht Substituenten;
- der Heteroaromat bis zu fünf Substituenten; und/oder
- die Reste R¹bis R⁴ bis zu acht Substituenten
auf, die unabhängig voneinander ausgewählt sind aus der Gruppe von Fluor, Chlor, CF₃, OH, NO₂, CN, R⁵, O-R⁵, CHO, CO-R⁵, COOH, COO-R⁵, OCO-R⁵, SiR⁵₃, NH₂, NH-R⁵, N-R⁵₂, SO-R⁵, SO₂-R⁵, SO₃H, SO₃-R⁵, CONH₂, NHCOH, NHCO-R⁵, NHCOO-R⁵, CHCH-CO₂-Alkyl-(C₁-C₈), PO-R⁵₂, P-R⁵₂, PO₃H₂, PO(O-Alkyl-(C₁-C₆))₂ und CHCHCO₂H; wobei R⁵ einen Alkylrest mit 1 bis 8 Kohlenstoffatomen oder einen Arylrest mit 6 bis 10 Kohlenstoffatomen, wie z. B. Phenyl, darstellt.

In der Formel (IVb) kann die Kohlenstoffbrücke E bis zu sieben Substituenten aufweisen, die unabhängig voneinander ausgewählt sind aus der Gruppe von (C₁-C₄)-Alkyl, O-Alkyl-(C₁-C₄), OH und Ar, wobei Ar die gleiche Bedeutung hat wie in Formel (I).

Ebenso ist es möglich, daß in der Formel (IIIa) Q₁ und Q₂ unabhängig voneinander einen (C₁-C₄)-Alkyl-, (C₁-C₄)-Alkoxy-, (C₆-C₁₀)-Aryl- und/oder (C₆-C₁₀)-Aryloxyrest darstellen, die durch mindestens ein Halogenatom oder einen (C₁-C₄)-Alkoxy- oder (C₁-C₄)-Alkylrest substituiert sind; oder daß Q₁ und Q₂ in der Formel (IIIa) zusammen eine Alkylendioxy- oder Alkylengruppe mit ein bis vier Kohlenstoffatomen darstellen, die gegebenenfalls durch bis zu vier (C₁-C₄)-Alkyl- und/oder (C₆-C₁₀)-Arylreste substituiert ist.

Die Reste Ar und Ar' können unabhängig voneinander einen Heteroaromaten darstellen, wobei an den heteroaromatischen Ring bis zu vier weitere aromatische, heteroaromatische und/oder aliphatische Ringe ankondensiert sind.
Das erfindungsgemäße Verfahren ist besonders geeignet für die Synthese von Biarylen, worin Ar und Ar' für einen substituierten Phenyl-, Naphthyl-, Anthryl-, Phenanthryl-, Biphenyl-Rest und/oder einen fünf-, sechs- oder siebengliedrigen Heteroaromaten mit gegebenenfalls Stickstoff-, Sauerstoff- oder Schwefelatomen im Ring stehen. Im Falle der Heteroaromaten sind besonders Heteroaromaten wie substituierte Pyridine, Pyrimidine, Oxazole, Imidazole, Pyrazine, Chinoline, Indole, Furane, Benzofurane oder/und Thiophene bevorzugt.

Das erfindungsgemäße Verfahren hat sich insbesondere für die Herstellung von Verbindungen der Formel (I) bewährt, in der die Reste Ar und Ar' unabhängig voneinander bis zu 5 Substituenten aufweisen, ausgewählt aus der Gruppe von Alkyl-(C₁-C₈), O-Alkyl-(C₁-C₈), OCO-Alkyl-(C₁-C₈), N-Alkyl₂-(C₁-C₈), Phenyl, Aryl, Fluor, Chlor, NO₂, CN, COOH, CHO, SO₂-Alkyl-(C₁-C₄), NH-Alkyl-(C₁-C₈), COO-Alkyl-(C₁-C₈), CONH₂, CONH-Alkyl-(C₁-C₈), CO-Alkyl-(C₁-C₈), CO-Phenyl und PO-Phenyl₂.

Die Umsetzung findet im allgemeinen in Gegenwart mindestens eines Lösungsmittels statt, ausgewählt aus der Gruppe von Wasser, aliphatischen Ethem, aromatischen oder aliphatischen Kohlenwasserstoffen, Alkoholen, Estern, aromatischen oder aliphatischen Nitrilen und dipolar aprotischen Lösungsmitteln wie Dialkylsulfoxiden, N,N-Dialkylamiden aliphatischer Carbonsäuren oder alkylierten Lactamen.
Das Lösungsmittel ist bevorzugt THF, Dioxan, Diethylether, Diglyme, MTBE, DME, Acetonitril, Toluol, Xylole, Anisol, Ethylacetat, Methanol, Ethanol, Butanol, Ethylenglycol, Ethylencarbonat, Propylencarbonat, Dimethylsulfoxid, N,N-Dimethylacetamid, N,N-Dimethylformamid oder N-Methylpyrrolidon.

Da bei der Reaktion eine Säure gebildet wird, ist es vorteilhaft, die entstehende Säure durch Zusatz einer Base abzufangen. Hierfür geeignet sind
- primäre, sekundäre und tertiäre Amine wie Alkylamine, Dialkylamine, Trialkylamine, die alicyclisch oder offenkettig sein können;
- Alkali- und Erdalkalisalze aliphatischer oder/und aromatischer Carbonsäuren, wie Acetate, Propionate, Benzoate, insbesondere deren Carbonate, Hydrogencarbonate, Phosphate, Hydrogenphosphate oder/und Hydroxide; sowie
- Metallalkoxide, insbesondere Alkali- oder Erdalkalialkoxide, wie Natriummethanolat, Kaliummethanolat, Natriumethanolat, Magnesiummethanolat und Calciumethanolat.

Bevorzugterweise ist die Base ein Carbonat, Hydroxid oder Phosphat von Lithium, Natrium, Kalium, Calcium, Magnesium oder Cäsium.

Die eingesetzte Base kann neben der Neutralisation der entstehenden Säure auch durch eine Aktivierung der Arylboronsäure zu anionischen Boranatspezies den Reaktionsverlauf positiv beeinflussen. Neben den oben genannten Basen kann eine solche Aktivierung auch durch Zusatz von Fluoridsalzen wie beispielsweise CaF₂, NaF, KF, LiF, CsF oder (C₁-C₈)-Alkyl₄NF erreicht werden.

Die eingesetzten Palladiumkatalysatoren der allgemeinen Formel (IVa) oder (IVb) werden in der Regel in situ aus mindestens einem Palladium(II)salz oder einer Palladium(0)verbindung und den entsprechenden Phosphitliganden erzeugt. Das Palladium(II)salz ist vorzugsweise ausgewählt aus der Gruppe von Palladium(II)acetat, Palladium(II)chlorid, Palladium(II)bromid, Lithiumtetrachloropalladat(II), Palladium(II)acetylacetonat, Palladium(II)-propionat, Palladium(II)chlorid-bis-(acetonitril), Palladium(II)-bis(triphenylphosphan)-dichlorid und Palladium(II)chloridbis(benzonitril).
Die Palladium(0)verbindung ist insbesondere ausgewählt aus der Gruppe der Palladium(0)-dibenzylidenaceton-Komplexe, Palladium(0)-tetrakis(triphenylphosphit), Palladium(0)-tetrakis(triethylphosphit), Palladium(0)-tetrakis(triphenylphosphan), Palladium(0)-bis(tri-o-tolylphosphan), Palladium(0)tricyclohexylphosphan-diallylether-Komplex und Palladium(0)bis(tricyclo-hexylphosphan).

Bei Einsatz von Chloraromaten, Bromaromaten, Aryltriflaten und/oder Arylmesylaten sowie verwandten Ausgangsprodukten ist es mitunter vorteilhaft, einen Co-Katalysator zum Palladium-Phosphit-Katalysator zuzusetzen. Dieser Co-Katalysator ist ausgewählt aus der Gruppe von Halogensalzen, insbesondere ein Halogenid der Alkalielemente oder/und Erdalkalielemente, ein Ammoniumhalogenid oder/und ein Phosphoniumhalogenid, vorzugsweise ein Bromid oder/und Chlorid.
Besonders bevorzugt sind die Halogensalze LiBr, LiCl, NaBr, KBr, CsBr, Bu₄NCl, Bu₄NBr, Benzyltrimethylammoniumbromid, Trioctylmethylammoniumbromid oder Tetraphenylphosphoniumbromid.
Der Co-Katalysator wird bezogen auf die Verbindung der Formel (II) üblicherweise in einer Menge von 0,001 mol% bis 100 mol%, insbesondere von 0,01 bis 50 mol%, eingesetzt.
Bei verfahrenstechnischen Vorteilen kann die Reaktion auch im Co-Katalysator als Lösungsmittel (Salzschmelze) durchgeführt werden.

Die Umsetzung wird im allgemeinen bei einer Temperatur zwischen 20 und 200° C, vorzugsweise zwischen 60 und 180° C, insbesondere zwischen 80 und 160° C, sowie bei einem Druck bis zu 100 bar, vorzugsweise bei einem Druck zwischen Normaldruck und 60 bar, durchgeführt.

Mit dem erfindungsgemäßen Verfahren können Tumover-Werte der Katalysatoren in der Größenordnung von 100.000 und mehr für Bromaromaten als Ausgangsmaterialien sowie von 10.000 und mehr für Chloraromaten realisiert werden. Diese Werte entsprechen den besten bekannten Ergebnissen, die mit Phosphanliganden erzielt wurden.

Aufgrund der Katalysatoraktivitäten und -stabilitäten ist es mit dem erfindungsgemäßen Verfahren erstmals möglich, extrem kleine Mengen an Katalysator zu verwenden, so daß die Katalysatorkosten im Vergleich zu den bekannten Suzuki-Reaktionen für den entsprechenden Prozeß nicht kostenlimitierend sind.
Bei dem erfindungsgemäßen Verfahren werden ausnahmsweise Gehalte an Katalysatoren von 1 bis 2 mol%, üblicherweise < 1 mol%, besonders bevorzugt < 0,2 mol% verwendet.
Darüberhinaus sind Phosphitliganden gegenüber den bisher verwendeten Phosphanliganden einfacher und kostengünstiger herzustellen, leichter modifizierbar sowie stabiler gegenüber Oxidationsreaktionen.

Die erfindungsgemäß hergestellten Biaryle werden unter anderem technisch eingesetzt als Zwischenprodukte für Pharmazeutika (ATII-Antagonisten) und Agrochemikalien, als Ligandvorstufe für Metallocenkatalysatoren, optische Aufheller und Bausteine für Polymere.

Die nachstehenden Beispiele dienen der Erläuterung des erfindungsmäßigen Verfahrens, ohne es darauf zu beschränken.

### Beispiele:

### Allgemeine Arbeitsvorschrift :

In einem Druckrohr (Fa. Aldrich) werden unter einer Argon-Atmosphäre 8,2 mmol Arylhalogenid, 9 mmol Phenylboronsäure, 10 mmol Base, gegebenenfalls eine geeignete Menge an Co-Katalysator, sowie eine geeignete Menge an Phosphit und Palladium(II)acetat und 500 mg Diethylenglycoldi-*n*-butylether (als interner Standard für die GC-Analytik) zu 8 ml trockenem Toluol gegeben. Das Rohr wird anschließend verschlossen und in ein 120° C ( bzw. 140° C in Ansatz Nr. 20) heißes Siliconölbad gegeben.
Nach einer Dauer von 20 h wird es auf Raumtemperatur abgekühlt. Die Feststoffe werden in 10 ml CH₂Cl₂ und 10 ml 1 *n* Natronlauge gelöst. Die organische Phase wird gaschromatographisch analysiert. Die Produkte werden durch Destillation, Kristallisation aus Methanol/Aceton-Mischungen oder säulenchromatographisch (Kieselgel, Hexan/Ethylacetat-Mischungen) isoliert.

In der folgenden Tabelle 1 sind die verwendetene Edukte sowie die erzielte Ausbeute und Turnover-Werte (TON) angegeben.

**Tabelle 1:**

| Übersicht über die erfindungsgemäßen Beispiele | | | | | | | | |
|---|---|---|---|---|---|---|---|---|
| Nr. | Halogenaromat | Base | Co-Kat. (mol%) | Phosphit | Pd [mol%] | P/Pd | GC-Ausbeute [%] | TON |
| 1 | 4-Chlorbenzotrifluorid | Na₂CO₃ | - | P(OⁱPr)₃ | 1 | 50 | 52 | 52 |
| 2 | 4-Chlorbenzotrifluorid | Na₂CO₃ | CsF (10) | P(OⁱPr)₃ | 1 | 50 | 58 | 58 |
| 3 | 4-Chlorbenzotrifluorid | Na₂CO₃ | CaF₂ (10) | P(OⁱPr)₃ | 1 | 50 | 69 | 69 |
| 4 | 4-Chlorbenzotrifluorid | Na₂CO₃ | H₂O (50) | P(OⁱPr)₃ | 1 | 50 | 70 | 70 |
| 5 | 4-Chlorbenzotrifluorid | K₃PO₄ | - | P(OⁱPr)₃ | 1 | 50 | 61 | 61 |
| 6 | 4-Chlorbenzotrifluorid | K₃PO₄ | - | P(OⁱPr)₃ | 0,001 | 50 | 15 | 15.000 |
| 7 | 4-Chlorbenzotrifluorid | NaOH | - | P(OⁱPr)₃ | 1 | 50 | 71 | 71 |
| 8 | 4-Chlorbenzotrifluorid | Na₂CO₃ | H₂O (50) | P(O-2,4-^{t}Bu₂C₆H₃)₃ | 1 | 10 | 70 | 70 |
| 9 | 4-Chlorbenzotrifluorid | Na₂CO₃ | H₂O (50) | P(OEt)₃ | 1 | 50 | 43 | 43 |
| 10 | 3-Chlorbenzotrifluorid | Na₂CO₃ | - | P(OⁱPr)₃ | 1 | 50 | 52 | 52 |
| 11 | 4-Chlornitrobenzol | Na₂CO₃ | - | P(O-2,4-^{t}Bu₂C₆H₃)₃ | 0,1 | 10 | 57 | 570 |
| 12 | 4-Chloracetophenon | Na₂CO₃ | - | P(O-2,4-^{t}Bu₂C₆H₃)₃ | 0,1 | 10 | 53 | 530 |
| 13 | 2-Chlorbenzonitril | Na₂CO₃ | - | P(OⁱPr)₃ | 1 | 10 | 80 | 80 |
| 14 | 4-Bromfluorbenzol | Na₂CO₃ | - | P(O-2,4-^{t}Bu₂C₆H₃)₃ | 0,1 | 10 | 72 | 720 |
| 15 | 4-Bromfluorbenzol | Na₂CO₃ | - | P(O-2,4-^{t}Bu₂C₆H₃)₃ | 0,0001 | 10 | 24 | 240.000 |
| 16 | 4-Bromtoluot | Na₂CO₃ | - | P(O-2,4-^{t}Bu₂C₆H₃)₃ | 0,1 | 10 | 86 | 860 |
| 17 | Brombenzol | Na₂CO₃ | - | P(O-2,4-^{t}Bu₂C₆H₃)₃ | 0,1 | 10 | 87 | 870 |
| 18 | 2-Brom-6methoxynaphthalin | Na₂CO₃ | - | P(O-2,4-^{t}Bu₂C₆H₃)₃ | 0,1 | 10 | 69 | 690 |
| 19 | Chlorbenzol | NaOH | - | P(O-2,4-^{t}Bu₂C₆H₃)₃ | 1 | 10 | 54 | 54 |
| 20 | 4-Chlortoluol | NaOH | - | P(O-2,4-^{t}Bu₂C₆H₃)₃ | 1 | 10 | 45 | 45 |
| 21 | 2-Brom-6-methoxynaphthalin | NaOH | - | P(OiPr)₃ | 0,0001 | 100 | 82 | 820.000 |
| 22 | 4-Bromfluorbenzol | NaOH | - | P(O-2,4-^{t}Bu₂C₆H₃)₃ | 0,0001 | 100 | 66 | 660.000 |
| 23 | Brombenzol | NaOH | - | P(O-2,4-^{t}Bu₂C₆H₃)₃ | 0,0001 | 100 | 85 | 850.000 |
| 24 | 4-Bromtoluol | NaOH | - | P(O-2,4-^{t}Bu₂C₆H₃)₃ | 0,0001 | 100 | 69 | 690.000 |

Die Beispiele belegen, daß es bei dem erfindungsgemäßen Verfahren möglich ist, Ausbeuten von mindestens 43 % und in vielen Fällen von mehr als 70 % bei erhöhten Tumover-Werten zu erreichen oder sehr hohe Turnover-Werte bei gewissen Ausbeuten.

## Patentansprüche

1. Verfahren zur Herstellung mono-, bi- und/oder polyfunktioneller Biaryle der allgemeinen Formel (I)
Ar - Ar' (I)
in der Ar und Ar' unabhängig voneinander
• einen aromatischen Rest mit bis zu 14 Kohlenstoffatomen oder
• einen Heteroaromaten darstellen, ausgewählt aus der Gruppe der fünf-. sechs- oder siebengliedrigen Ringe mit mindestens einem Stickstoff-, Sauerstoff- und/oder Schwefelatom im Ring;
durch Umsetzung von Halogenarometen der allgemeinen Formel (II)
Ar - X (II)
mit Borverbindungen der allgemeinen Formel (IIIa), (IIIb) und/oder (IIIc) 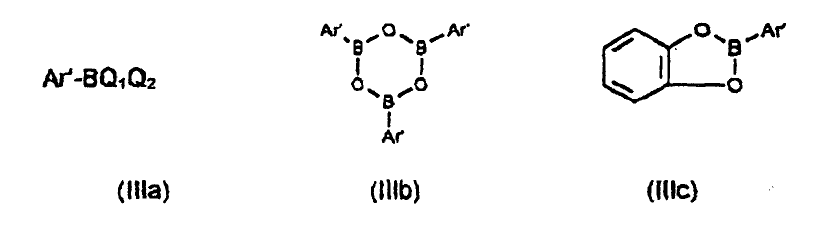 wobei in den Formeln (II), (IIIa), (IIIb) und (IIIc)
• Ar und Ar' die gleiche Bedeutung haben wie in Formel (I);
• X ausgewählt ist aus der Gruppe von Chlor, Brom, Iod, OSO₂CF₃, OSO₂Aryl-(C₆-C₁₀), OSO₂Alkyl-(C₁-C₈) und N₂⁺Y⁻, wobei Y ein Chlor-, Brom- oder Iodatom oder ein Tetrafluoroborat- oder Tetraphenylboratanion darstellt;
• Q₁ und Q₂ unabhängig voneinander ausgewählt sind aus der Gruppe von OH, Fluor, Chlor, Brom, Iod, Alkyl-(C₁-C₄), Aryl-(C₈-C₁₀), Alkoxy-(C₁-C₄) und Aryloxy-(C₈-C₁₀);
in Gegenwart mindestens eines Palladiumkomplexes der allgemeinen Formel (IVa) oder (IVb) 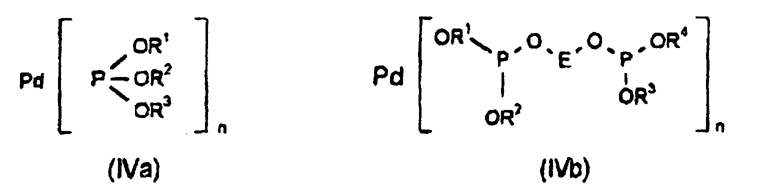 in denen
• die Reste R¹ bis R⁴ unabhängig voneinander einen (C₁-C₁₈)-Alkylrest oder einen der zuvor beschriebenen Reste Ar darstellen;
• E eine Kohlenstoffbrücke mit zwei bis sieben Kohlenstoffatomen darstellt; und
• n und m unabhängig voneinander eine ganze Zahl von 1 bis 4 darstellen und wobei die Palladiumkomplexe der allgemeinen Formel (IVa) oder (IVb) in situ aus mindestens einem Palladium(II)salz oder einer Palladium(0)verbindung und dem entsprechenden Phosphitliganden erhalten werden und das molare Verhältnis der eingesetzten Palladium(0)verbindung oder des Palladium (II)salzes zu eingesetztem Phosphitligand zwischen 1 : 5 und 1 : 200 liegt.

2. Verfahren nach Anspruch 1, **dadurch gekennzeichnet, daß**
• die aromatischen Reste Ar und Ar' bis zu acht Substituenten;
• der Heteroaromat bis zu fünf Substituenten; und/oder
• die Reste R¹bis R⁴ bis zu acht Substituenten
aufweisen, die unabhängig voneinander ausgewählt sind aus der Gruppe von Fluor, Chlor, CF₃, OH, NO₂, CN, R⁵, O-R⁵, CHO, CO-R⁵, COOH, COO-R⁵, OCO-R⁵, SiR⁵₃, NH₂, NH-R⁵, N-R⁵₂, SO-R⁵, SO₂-R⁵, SO₃H, SO₃-R⁵, CONH₂, NHCOH, NHCO-R⁵, NHCOO-R⁵, CHCH-CO₂-Alkyl-(C₁-C₈), PO-R⁵₂, P-R⁵₂, PO₃H₂, PO(O-Alkyl-(C₁-C₆))₂ und CHCHCO₂H; wobei R⁵ einen Alkylrest mit 1 bis 8 Kohlenstoffatomen oder einen Arylrest mit 6 bis 10 Kohlenstoffatomen, wie z.B. Phenyl, darstellt.

3. Verfahren nach Anspruch 1 oder 2, **dadurch gekennzeichnet, daß** die Kohlenstoffbrücke E bis zu sieben Substituenten aufweist, die unabhängig voneinander ausgewählt sind aus der Gruppe von (C₁-C₄)-Alkyl, O-Alkyl-(C₁-C₄), OH und Ar, wobei Ar die gleiche Bedeutung hat wie in Formel (I).

4. Verfahren nach einem der Ansprüche 1 bis 3, **dadurch gekennzeichnet, daß** in der Formel (IIIa) Q₁ und Q₂ unabhängig voneinander einen (C₁-C₄)-Alkyl-, (C₁-C₄)-Alkoxy-, (C₆-C₁₀)-Alryl- und/oder (C₆-C₁₀)-Aryloxyrest darstellen, die durch mindestens ein Halogenatom oder einen (C₁-C₄)-Alkoxy- oder (C₁-C₄)-Alkylrest substituiert sind.

5. Verfahren nach einem der Ansprüche 1 bis 3, **dadurch gekennzeichnet, daß** in der Formel (IIIa) Q₁ und Q₂ zusammen eine Alkylendioxy- oder Alkylengruppe mit ein bis vier Kohlenstoffatomen darstellen, die gegebenenfalls durch bis zu vier (C₁-C₄)-Alkyl- und/oder (C₈-C₁₀)-Arylreste substituiert ist.

6. Verfahren nach einem der vorhergehenden Ansprüche, **dadurch gekennzeichnet, daß** die Reste Ar und Ar' unabhängig voneinander einen Heteroaromaten darstellen, wobei an den heteroaromatischen Ring bis zu vier weitere aromatische, heteroaromatische und/oder aliphatische Ringe ankondensiert sind.

7. Verfahren nach einem der vorhergehenden Ansprüche, **dadurch gekennzeichnet, daß** die Reste Ar und Ar' unabhängig voneinander einen substituierten Phenyl-, Naphthyl-, Anthryl-, Phenanthryl-, Biphenyl-, Pyridin-, Pyrimidin-, Oxazol-, Imidazol-, Pyrazin-, Chinolin-, Indol-, Furan, Benzofuranoder Thiophenrest darstellen.

8. Verfahren nach einem der vorhergehenden Ansprüche, **dadurch gekennzeichnet, daß** die Reste Ar und Ar' unabhängig voneinander bis zu 5 Substituenten aufweisen, ausgewählt aus der Gruppe von Alkyl-(C₁-C₈), O-Alkyl-(C₁-C₈), OCO-Alkyl-(C₁-C₈), N-Alkyl₂-(C₁-C₈), Phenyl, Aryl, Fluor, Chlor, NO₂, CN, COOH, CHO, SO₂-Alkyl-(C₁-C₄), NH-Alkyl-(C₁-C₈), COO-Alkyl-(C₁-C₈), CONH₂, CONH-Alkyl-(C₁-C₈), CO-Alkyl-(C₁-C₈), CO-Phenyl und PO-Phenyl₂.

9. Verfahren nach einem der vorhergehenden Ansprüche, **dadurch gekennzeichnet, daß** die Umsetzung in Gegenwart mindestens eines Lösungsmittels durchgeführt wird, ausgewählt aus der Gruppe von Wasser, aliphatischen Ethem, aromatischen oder aliphatischen Kohlenwasserstoffen, Alkoholen, Estern, aromatischen oder aliphatischen Nitrilen und dipolar aprotischen Lösungsmitteln wie Dialkylsulfoxiden, N,N-Dialkylamiden aliphatischer Carbonsäuren oder alkylierten Lactamen.

10. Verfahren nach Anspruch 9, **dadurch gekennzeichnet, daß** das Lösungsmittel THF, Dioxan, Diethylether, Diglyme, MTBE, DME, Acetonitril, Toluol, Xylol, Anisol, Ethylacetat, Methanol, Ethanol, Butanol, Ethylenglycol, Ethylencarbonat, Propylencarbonat, Dimethylsulfoxid, N,N-Dimethylacetamid, N,N-Dimethylformamid oder N-Methylpyrrolidon ist.

11. Verfahren nach einem der vorhergehenden Ansprüche, **dadurch gekennzeichnet, daß** die Umsetzung in Gegenwart mindestens einer Base durchgeführt wird, ausgewählt aus der Gruppe der
• primären, sekundären und tertiären Amine wie Alkylamine, Dialkylamine, Trialkylamine, die alicyclisch oder offenkettig sein können;
• Alkali- und Erdalkalisalze aliphatischer oder/und aromatischer Carbonsäuren, wie Acetate, Propionate, Benzoate, insbesondere deren Carbonate, Hydrogencarbonate, Phosphate, Hydrogenphosphate oder/und Hydroxide;
• Metallalkoxide, insbesondere Alkali- oder Erdalkalialkoxide, wie Natriummethanolat, Kaliummethanolat, Natriumethanolat, Magnesiummethanolat und Calciumethanolat.

12. Verfahren nach Anspruch 11, **dadurch gekennzeichnet, daß** die Base ein Carbonat, Hydroxid oder Phosphat von Lithium, Natrium, Kalium, Calcium, Magnesium oder Cäsium ist.

13. Verfahren nach einem der vorhergehenden Ansprüche, **dadurch gekennzeichnet, daß** die Umsetzung in Gegenwart mindestens eines Fluoridsalzes durchgeführt wird, das insbesondere ausgewählt ist aus der Gruppe von CaF₂, NaF, KF, LiF, CsF und (C₁-C₈)-Alkyl₄NF.

14. Verfahren nach Anspruch 1, **dadurch gekennzeichnet, daß** die Palladium(0)verbindung ausgewählt ist aus der Gruppe der Palladium(0)-dibenzylidenaceton-Komplexe, Palladium(0)-tetrakis(triphenylphosphit), Palladium(0)-tetrakis(triethylphosphit), Palladium(0)-tetrakis(triphenyl phosphan), Palladium(0)-bis(tri-o-tolylphosphan), Palladium(0)tricyclohexyl phosphan-diallylether-Komplex und Palladium(0)bis(tricyclo-hexylphosphan).

15. Verfahren nach Anspruch1, **dadurch gekennzeichnet, daß** das Palladium(II) salz ausgewählt ist aus der Gruppe von Palladium(II)acetat, Palla dium(II)chlorid, Palladium(II)bromid, Lithiumtetrachloropalladat(II), Palladium(II) acetylacetonat, Palladium(II)-propionat, Palladium(II)chlorid-bis(acetonitril), Palladium(II)-bis(triphenylphosphan)-dichlorid und Palladium(II)chloridbis(benzonitril).

16. Verfahren nach einem der vorhergehenden Ansprüche, **dadurch gekennzeichnet, daß** die Umsetzung in Gegenwart mindestens eines Co-Katalysators durchgeführt wird, ausgewählt aus der Gruppe von Halogensalzen, insbesondere ein Halogenid der Alkalielemente oder/und Erdalkalielemente, ein Ammoniumhalogenid oder/und ein Phosphoniumhalogenid, vorzugsweise ein Bromid oder/und Chlorid.

17. Verfahren nach Anspruch 16, **dadurch gekennzeichnet, daß** das Halogensalz LiBr, LiCl, NaBr, KBr, CsBr, Bu₄NCl, Bu₄NBr, Benzyltrimethylammoniumbromid, Trioctylmethylammoniumbromid oder Tetraphenylphosphoniumbromid ist.

18. Verfahren nach Anspruch 16 oder 17, **dadurch gekennzeichnet, daß** der Co-Katalysator in einer Menge von 0,001 mol% bis 100 mol%, insbesondere von 0.01 bis 50 mol%, bezogen auf die Verbindung der Formel (II) eingesetzt wird.

19. Verfahren nach einem der Ansprüche 16 bis 18, **dadurch gekennzeichnet, daß** die Umsetzung in Gegenwart des geschmolzenen Co-Katalysators (Salz schmelze) durchgeführt wird.

20. Verfahren nach einem der vorhergehenden Ansprüche, **dadurch gekennzeichnet, daß** die Umsetzung bei einer Temperatur zwischen 20 und 200° C, vorzugsweise zwischen 60 und 180° C, insbesondere zwischen 80 und 160° C, durchgeführt wird.

21. Verfahren nach einem der vorhergehenden Ansprüche, **dadurch gekennzeichnet, daß** die Umsetzung bei einem Druck bis zu 100 bar, vorzugsweise bei einem Druck zwischen Normaldruck und 60 bar, durchgeführt wird.

## Claims

1. A process for preparing monofunctional, bifunctional and/or polyfunctional biaryls of the formula (I)
Ar - Ar' (I)
where Ar and Ar' are each, independently of one another,
• an aromatic radical having up to 14 carbon atoms or
• a heteroaromatic selected from the group consisting of five-, six- or seven-membered rings having at least one nitrogen, oxygen and/or sulfur atom in the ring;
by reacting haloaromatics of the formula (II)
Ar - X (II)
with boron compounds of the formula (IIIa), (IIIb) and/or (IIIc) 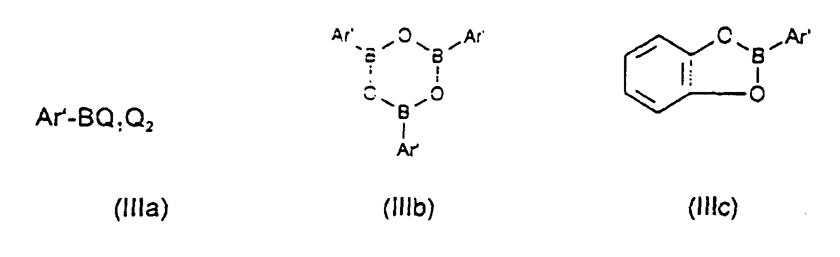 where, in the formulae (II), (IIIa), (IIIb) and (IIIc),
• Ar and Ar' are as defined for formula (I);
• X is selected from the group consisting of chlorine, bromine, iodine, OSO₂CF₃, OSO₂aryl-(C₆-C₁₀), OSO₂alkyl-(C₁-C₈) and N₂⁺Y⁻, where Y is a chlorine, bromine or iodine atom or a tetrafluoroborate or tetraphenylborate anion;
• Q₁ and Q₂ are selected independently from the group consisting of OH, fluorine, chlorine, bromine, iodine, alkyl-(C₁-C₄), aryl- (C₆-C₁₀), alkoxy-(C₁-C₄) and aryloxy-(C₆-C₁₀);
in the presence of at least one palladium complex of the formula (IVa) or (IVb), 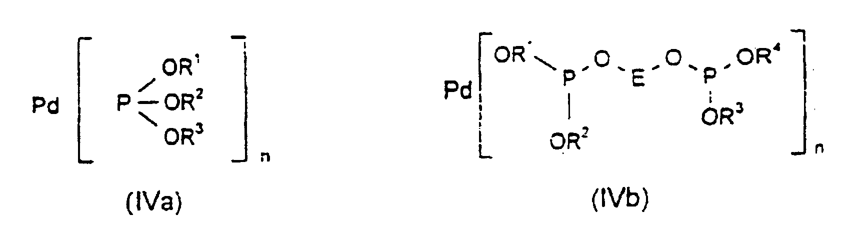 where
• the radicals R¹ to R⁴ are each, independently of one another, a (C₁-C₁₈)-alkyl radical or one of the above-described radicals Ar;
• E is a carbon bridge having from two to seven carbon atoms; and
• n and m are each, independently of one another, an integer from 1 to 4, and where the palladium complexes of the formula (IVa) or (IVb) are obtained in situ from at least one palladium(II) salt or a palladium(0) compound and the corresponding phosphite ligand and the molar ratio of the palladium(0) compound used or the palladium(II) salt to phosphite ligand used is in the range from 1:5 to 1:200.

2. The process as claimed in claim 1, **characterized in that**
• the aromatic radicals Ar and Ar' have up to eight substituents;
• the heteroaromatic has up to five substituents; and/or
• the radicals R¹ to R⁴ have up to eight substituents
which are selected independently from the group consisting of fluorine, chlorine, CF₃, OH, NO₂, CN, R⁵, O-R⁵, CHO, CO-R⁵, COOH, COO-R⁵, OCO-R⁵, SiR⁵₃, NH₂, NH-R⁵, N-R⁵₂, SO-R⁵, SO₂-R⁵, SO₃H, SO₃-R⁵, CONH₂, NHCOH, NHCO-R⁵, NHCOO-R⁵, CHCH-CO₂-alkyl-(C₁-C₈), PO-R⁵₂, P-R⁵₂, PO₃H₂, PO(O-alkyl-(C₁-C₆))₂ and CHCHCO₂H; where R⁵ is an alkyl radical having from 1 to 8 carbon atoms or an aryl radical having from 6 to 10 carbon atoms, e.g. phenyl.

3. The process as claimed in claim 1 or 2, **characterized in that** the carbon bridge E has up to seven substituents selected independently from the group consisting of (C₁-C₄)-alkyl, O-alkyl-(C₁-C₄), OH and Ar, where Ar is as defined for formula (I).

4. The process as claimed in any of claims 1 to 3, **characterized in that** Q₁ and Q₂ in the formula (IIIa) are each, independently of one another, a (C₁-C₄)-alkyl, (C₁-C₄)-alkoxy, (C₆-C₁₀)-aryl or (C₆-C₁₀)-aryloxy radical which is substituted by at least one halogen atom or a (C₁-C₄)-alkoxy or (C₁-C₄)-alkyl radical.

5. The process as claimed in any of claims 1 to 3, **characterized in that** Q₁ and Q₂ in the formula (IIIa) are together an alkylenedioxy or alkylene group which has from one to four carbon atoms and may be substituted by up to four (C₁-C₄)-alkyl and/or (C₆-C₁₀)-aryl radicals.

6. The process as claimed in any of the preceding claims, **characterized in that** the radicals Ar and Ar' are each, independently of one another, a heteroaromatic in which up to four further aromatic, heteroaromatic and/or aliphatic rings are fused onto the heteroaromatic ring.

7. The process as claimed in any of the preceding claims, **characterized in that** the radicals Ar and Ar' are each, independently of one another, a substituted phenyl, naphthyl, anthryl, phenanthryl, biphenyl, pyridine, pyrimidine, oxazole, imidazole, pyrazine, quinoline, indole, furan, benzofuran or thiophene radical.

8. The process as claimed in any of the preceding claims, **characterized in that** the radicals Ar and Ar' each have, independently of one another, up to 5 substituents selected from the group consisting of alkyl-(C₁-C₈), O-alkyl-(C₁-C₈), OCO-alkyl-(C₁-C₈), N-alkyl₂-(C₁-C₈), phenyl, aryl, fluorine, chlorine, NO₂, CN, COOH, CHO, SO₂-alkyl-(C₁-C₄), NH-alkyl-(C₁-C₈), COO-alkyl-(C₁-C₈), CONH₂, CONH-alkyl-(C₁-C₈), CO-alkyl-(C₁-C₈), CO-phenyl and PO-phenyl₂.

9. The process as claimed in any of the preceding claims, **characterized in that** the reaction is carried out in the presence of at least one solvent selected from the group consisting of water, aliphatic ethers, aromatic and aliphatic hydrocarbons, alcohols, esters, aromatic and aliphatic nitriles and dipolar aprotic solvents such as dialkyl sulfoxides, N,N-dialkylamides of aliphatic carboxylic acids and alkylated lactams.

10. The process as claimed in claim 9, **characterized in that** the solvent is THF, dioxane, diethyl ether, diglyme, MTBE, DME, acetonitrile, toluene, xylene, anisole, ethyl acetate, methanol, ethanol, butanol, ethylene glycol, ethylene carbonate, propylene carbonate, dimethyl sulfoxide, N,N-dimethylacetamide, N,N-dimethylformamide or N-methylpyrrolidone.

11. The process as claimed in any of the preceding claims, **characterized in that** the reaction is carried out in the presence of at least one base selected from the group consisting of
• primary, secondary and tertiary amines such as alkylamines, dialkylamines, trialkylamines, which may be alicyclic or open-chain;
• alkali metal and alkaline earth metal salts of aliphatic and aromatic carboxylic acids such as acetates, propionates, benzoates, in particular their carbonates, hydrogencarbonates, phosphates, hydrogenphosphates or/and hydroxides; and
• metal alkoxides, in particular alkali metal and alkaline earth metal alkoxides such as sodium methoxide, potassium methoxide, sodium ethoxide, magnesium methoxide and calcium ethoxide.

12. The process as claimed in claim 11, **characterized in that** the base is a carbonate, hydroxide or phosphate of lithium, sodium, potassium, calcium, magnesium or cesium.

13. The process as claimed in any of the preceding claims, **characterized in that** the reaction is carried out in the presence of at least one fluoride salt which is, in particular, selected from the group consisting of CaF₂, NaF, KF, LiF, CsF and (C₁-C₈)-alkyl₄NF.

14. The process as claimed in claim 1, **characterized in that** the palladium(0) compound is selected from the group consisting of palladium(0)-dibenzylideneacetone complexes, tetrakis(triphenyl phosphite)palladium(0), tetrakis(triethyl phosphite)-palladium(0), tetrakis(triphenylphosphine)palladium(0), bis(tri-o-tolylphosphine)palladium(0), the tricyclohexylphosphinepalladium(0) diallyl ether complex and bis(tricyclohexylphosphine)-palladium(0).

15. The process as claimed in claim 1, **characterized in that** the palladium(II) salt is selected from the group consisting of palladium(II) acetate, palladium(II) chloride, palladium(II) bromide, lithium tetrachloropalladate(II), palladium(II) acetylacetonate, palladium(II) propionate, bis-(acetonitrile)palladium(II) chloride, bis(tri-phenylphosphine)palladium(II) dichloride and bis(benzonitrile)palladium(II) chloride.

16. The process as claimed in any of the preceding claims, **characterized in that** the reaction is carried out in the presence of at least one cocatalyst selected from the group consisting of halogen salts, in particular a halide of the alkali metals or/and alkaline earth metals, an ammonium halide or/and a phosphonium halide, preferably a bromide or/and chloride.

17. The process as claimed in claim 16, **characterized in that** the halogen salt is LiBr, LiCl, NaBr, KBr, CsBr, Bu₄NCl, Bu₄NBr, benzyltrimethylammonium bromide, trioctylmethylammonium bromide or tetraphenylphosphonium bromide.

18. The process as claimed in claim 16 or 17, **characterized in that** the cocatalyst is used in an amount of from 0.001 mol% to 100 mol%, in particular from 0.01 to 50 mol%, based on the compound of the formula (II).

19. The process as claimed in any of claims 16 to 18, **characterized in that** the reaction is carried out in the presence of the molten cocatalyst (salt melt).

20. The process as claimed in any of the preceding claims, **characterized in that** the reaction is carried out at a temperature of from 20 to 200°C, preferably from 60 to 180°C, in particular from 80 to 160°C.

21. The process as claimed in any of the preceding claims, **characterized in that** the reaction is carried out at a pressure of up to 100 bar, preferably at a pressure in the range from atmospheric pressure to 60 bar.

## Revendications

1. Procédé pour la préparation de biaryles mono-, bi- et/ou polyfonctionnels de formule générale
**Ar - Ar' (I)**
dans laquelle Ar et Ar' représentent indépendamment l'un de l'autre
- un reste aromatique présentant jusqu'à 14 atomes de carbone,
- un composé hétéroaromatique pris dans le groupe des cycles à 5, 6 ou 7 chaînons comprenant au moins un atome d'azote, d'oxygène et/ou de soufre dans le cycle ;
par réaction d'un halogénoaromatique de formule générale (II)
**Ar - X (II)**
sur un composé de bore de formules générales (IIIa), (IIIb) et/ou (IIIc) 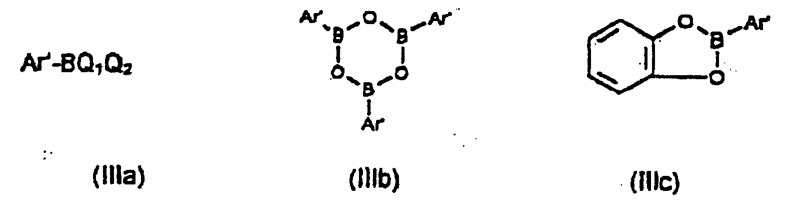 dans les formules (II), (IIIa), (IIIb) et (IIIc)
- Ar et Ar' possèdent les mêmes significations qu'à la formule (I) ;
- X est pris parmi les groupes chlore, brome, iode, OSO₂CF₃, OSO₂-(aryle en C₆-C₁₀), OSO₂-(alkyle en C₁-C₈) et N₂⁺Y⁻, Y représente un atome de chlore, de brome ou d'iode ou un anion tétrafluoroborate ou tétraphénylborate ;
- Q₁ et Q₂ indépendamment l'un de l'autre sont pris parmi les groupes OH, fluor, chlore, brome, iode, alkyle en C₁-C₄, aryle en C₈-C₁₀, alkoxy en C₁-C₄ et aryloxy en C₈-C₁₀ ;
en présence d'au moins un complexe de palladium de formule générale (IVa) ou (IVb) 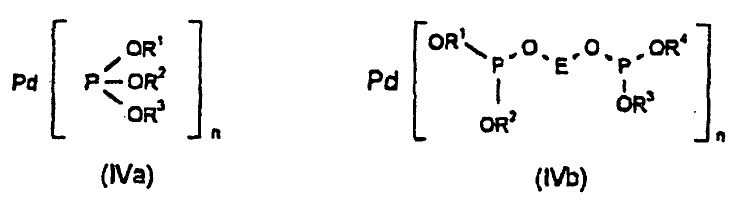 dans lesquels
- les restes R¹ à R⁴ représentent indépendamment l'un de l'autre un reste alkyle en C₁-C₁₈ ou un reste Ar décrit auparavant ;
- E représente une liaison carbonée de 2 à 7 atomes de carbone ; et
- n et m indépendamment l'un de l'autre sont un nombre de 1 à 4,
et on obtient les complexes de palladium de formule générale (IVa) ou (IVb) in situ à partir d'au moins un sel de palladium(II) ou d'un composé de palladium(0) et le ligand phosphite et le rapport molaire du composé de palladium (0) ou du sel de palladium(II) utilisé au ligand phosphite est compris entre 1:5 et 1:200.

2. Procédé selon la revendication 1, **caractérisé en ce que**
- les restes aromatiques Ar et Ar' présentent jusqu'à huit substituants ;
- l'hétéroaromatique présente jusqu'à cinq substituants ; et/ou
- les restes R¹ à R⁴ présentent jusqu'à huit substituants,
qui sont pris indépendamment parmi les groupes comprenant fluor, chlore, CF₃, OH, NO₂, CN, R⁵, O-R⁵, CHO, CO-R⁵, COOH, COO-R⁵, OCO-R⁵, SiR₃⁵, NH₂, NH-R⁵, N-R⁵₂, SO-R⁵, SO₂-R⁵, SO₃H, SO₃R⁵, CONH₂, NHCOH, NHCO-R⁵, NHCOO-R⁵, CHCH-CO₂-(alkyle en C₁-C₈), PO-R⁵₂, P-R⁵₂, PO₃H₂, PO(alkyle en C₁-C₆)₂ et CHCHCO₂H ; R⁵ représente un reste alkyle présentant 1 à 8 atomes de carbone ou un reste aryle présentant 6 à 10 atomes de carbone, comme par exemple phényle.

3. Procédé selon les revendications 1 ou 2, **caractérisé en ce que** la liaison carbonée E présente jusqu'à 7 substituants, qui sont pris indépendamment l'un de l'autre dans le groupe comprenant alkyle en C₁-C₄, O-alkyle en C₁-C₄, OH et Ar, Ar possédant la même signification qu'à la formule (I).

4. Procédé selon les revendications 1 à 3, **caractérisé en ce que** à la formule (IIIa) Q₁ et Q₂ représentent indépendamment l'un de l'autre un reste alkyle en C₁-C₄, alkoxy en C₁-C₄, aryle en C₆-C₁₀ et/ou aryloxy en C₆-C₁₀, qui sont substitués par au moins un atome d'halogène ou un reste alkoxy en C₁-C₄ ou alkyle en C₁-C₄.

5. Procédé selon les revendications 1 à 3, **caractérisé en ce que**, à la formule (IIIa), Q₁ et Q₂ représentent conjointement un groupe alkylènedioxy ou alkylène présentant jusqu'à quatre atomes de carbone, qui sont substitués par au moins un atome d'halogène ou un reste alkoxy en C₁-C₄ et/ou aryle en C₆-C₁₀.

6. Procédé selon l'une des revendications précédentes, **caractérisé en ce que** les restes Ar et Ar' représentent indépendamment l'un de l'autre un hétéroaromatique, le cycle hétéroaromatique étant condensé sur jusqu'à quatre autres cycles aromatiques, hétéroaromatiques et/ou aliphatiques.

7. Procédé selon l'une des revendications précédentes, **caractérisé en ce que** les restes Ar et Ar' indépendamment l'un de l'autre représentent un reste phényle, naphtyle, anthryle, phénanthryle, biphényle, pyridine, pyrimidine, oxazole, imidazole, pyrazine, quinoléine, indole, furanne, benzofuranne ou thiophène.

8. Procédé selon l'une des revendications précédentes, **caractérisé en ce que** les restes Ar et Ar' indépendamment l'un de l'autre présentent jusqu'à 5 substituants, pris parmi les groupes alkyle en C₁-C₈, O-(alkyle en C₁-C₈) OCO-(alkyle en C₁-C₈), N- (alkyle en C₁-C₈)₂, phényle aryle, fluor, chlore, NO₂, CN, COOH, CHO, SO₂-(alkyle en C₁-C₄), NH- (alkyle en C₁-C₈), COO-(alkyle en C₁-C₈), CONH₂, CONH-(alkyle en C₁-C₈), CO-(alkyle en C₁-C₆), CO-phényle et PO-phényle₂.

9. Procédé selon l'une des revendications précédentes, **caractérisé en ce que** la réaction est mise en oeuvre en présence d'au moins un solvant pris dans le groupe comprenant l'eau, des éthers aliphatiques, des hydrocarbures aromatiques ou aliphatiques, des alcools, des esters, des nitriles aromatiques ou aliphatiques et des solvants aprotiques dipolaires comme des dialkylsulfoxydes, des N,N-dialkylamides d'acides carboxyliques aliphatiques ou des lactames alkylés.

10. Procédé selon la revendication 9, **caractérisé en ce que** le solvant est THF, le dioxane, l'éther diéthylique, le diglyme, MTBE, DME, l'acétonitrile, le toluène, le xylène, l'anisole, l'acétate d'éthyle, le méthanol, l'éthanol, le butanol, l'éthylèneglycol, le carbonate d'éthylène, le carbonate de propylène, le diméthylsulfoxyde, le N,N-diméthylacétamide, le N,N-diméthylformamide ou la N-méthylpyrrolidone.

11. Procédé selon l'une des revendications précédentes, **caractérisé en ce que** la réaction est mise en oeuvre en présence d'au moins une base prise dans le groupe comprenant
- des amines primaires, secondaires et tertiaires, comme des alkylamines, des dialkylamines, des trialkylamines, qui peuvent être alicycliques ou à chaîne ouverte ;
- des sels de métaux alcalins et alcalino-terreux d'acides carboxyliques aliphatiques ou/et aromatiques, tels que des acétates, des propionates, des benzoates, en particulier des carbonates, des bicarbonates, des phosphates, des hydrogénophosphates ou/et hydroxydes ;
- des oxydes métalliques, en particulier des oxydes alcalins ou alcalino-terreux, comme le méthanolate de sodium, le méthanolate de potassium, le méthanolate de sodium, le méthanolate de magnésium et le méthanolate de calcium.

12. Procédé selon la revendication 11, **caractérisé en ce que** la base est un carbonate, un hydroxyde ou phosphate de lithium, de sodium, de potassium, de calcium, de magnésium ou de césium.

13. Procédé selon l'une des revendications précédentes, **caractérisé en ce que** la réaction est mise en oeuvre en présence d'au moins un sel fluorure, qui est notamment pris dans le groupe comprenant CaF₂, NaF, KF, LiF, CsF et (alkyl en C₁-C₈)₄NF.

14. Procédé selon la revendication 1, **caractérisé en ce que** le composé de palladium(0) est pris dans le groupe des complexes de dibenzylidénacétone de palladium(0), de tétrakis(triphénylphosphite) de palladium(0), de tétrakis(triéthylphosphite) de palladium(0), de tétrakis(triphénylphosphane) de palladium(0), de bis(tri-o-tolylphosphane) de palladium(0), le complexe d'éther tricyclohexylphosphane-diallylique et le bis(tricyclo-hexylphosphane) de palladium(0).

15. Procédé selon la revendication 1, **caractérisé en ce que** le sel de palladium(II) est pris dans le groupe comprenant l'acétate de palladium(II), le chlorure de palladium(II), le bromure de palladium(II), le tétrachloropalladate(II) de lithium, l'acétylacétonante de palladium(II), le propionate de palladium(II), le chlorure de bis(acétonitrile)-palladium(II), le dichlorure de bis(triphénylphosphane)-palladium(II) et le chlorure de bis(benzonitrile)-palladium(II).

16. Procédé selon l'une des revendications précédentes, **caractérisé en ce que** la réaction est mise en oeuvre en présence d'au moins un co-catalyseur, pris dans le groupe de sels halogénures, en particulier un halogénure des éléments alcalins et/ou éléments alcalino-terreux, un halogénure d'ammonium ou/et un halogénure de phosphonium, de préférence un bromure ou/et chlorure.

17. Procédé selon la revendication 16, **caractérisé en ce que** le sel halogénure est LiBr, LiCl, NaBr, KBr, CsBr, Bu₄NCl, Bu₄NBr, le bromure de benzyltriméthylammonium, le bromure de trioctylméthylammonium ou le bromure de tétraphénylphosphonium.

18. Procédé selon les revendications 16 ou 17, **caractérisé en ce qu'**on utilise le co-catalyseur dans une quantité de 0,001 % molaire à 100 % molaires, en particulier de 0,01 à 50 % molaires, par rapport au composé de formule (II).

19. Procédé selon l'une des revendications 16 à 18, **caractérisé en ce que** la réaction est mise en oeuvre en présence du co-catalyseur fondu (sel fondu).

20. Procédé selon l'une des revendications précédentes, **caractérisé en ce que** la réaction est mise en oeuvre à une température comprise entre 20 et 200°C, de préférence entre 60 et 180°C, en particulier entre 80 et 160°C.

21. Procédé selon l'une des revendications précédentes, **caractérisé en ce que** la réaction est mise en oeuvre sous une pression jusqu'à 100 bars, de préférence sous une pression comprise entre la pression normale et 60 bars.
